Europäisches Patentamt

European Patent Office

Office européen des brevets

(11) Veröffentlichungsnummer: **0 350 763**
**A1**

(12) EUROPÄISCHE PATENTANMELDUNG

(21) Anmeldenummer: 89112147.7

(22) Anmeldetag: 03.07.89

(51) Int. Cl.⁴: **C11C 3/12**

(30) Priorität: **11.07.88 DE 3823457**

(43) Veröffentlichungstag der Anmeldung:
**17.01.90 Patentblatt 90/03**

(84) Benannte Vertragsstaaten:
**ES**

(71) Anmelder: **Henkel Kommanditgesellschaft auf Aktien**
**Postfach 1100 Henkelstrasse 67**
**D-4000 Düsseldorf 1(DE)**

(72) Erfinder: **Göbel, Gerd, Dr.**
**Falkenweg 6**
**D-5000 Köln 40(DE)**
Erfinder: **Jeromin, Lutz, Dr.**
**Am Bandsbusch 88**
**D-4010 Hilden(DE)**
Erfinder: **Peukert, Eberhardt**
**Dürerweg 15**
**D-4010 Hilden(DE)**
Erfinder: **Wollman, Gerhard, Dr.**
**Kunibertstrasse 31**
**D-4010 Hilden(DE)**
Erfinder: **Carduck, Franz-Josef, Dr.**
**Landstrasse 18**
**D-5657 Haan(DE)**
Erfinder: **Bremus, Norbert**
**Turmstrasse 21**
**D-4018 Langenfeld(DE)**
Erfinder: **Kubersky, Hans-Peter, Dr.**
**Hasselstrasse 60**
**D-5650 Solingen 1(DE)**

(54) **Verfahren zum Hydrieren von Fettsäureestern, Fetten, Fettsäuren und Vorrichtung zum Durchführen des Verfahrens.**

(57) Die Erfindung betrifft ein Verfahren zum Hydrieren von Fettsäureestern, Fetten, Fettsäuren und eine Vorrichtung zum Durchführen dieses Verfahrens. Um die Raumzeitausbeute und die Katalysator-Standzeit zu erhöhen sowie die Zahl der Aufarbeitungsstufen zu verringern, wird vorgeschlagen, daß im Gegenstrom hydriert wird und daß die Stoffdurchflußrate und die Reaktorgeometrie so gewählt werden, daß ein Fluten des Reaktors nicht eintritt. Bei der Vorrichtung zum Durchführen des Verfahrens wird die Aufgabe dadurch gelöst, daß mindestens ein Hydrierreaktor mit mindestens einem ersten Einlaß (5) für Wasserstoff und einem Gasverteiler (2), mit mindestens einem zweiten Einlaß (6) für den zu hydrierenden Einsatz mit einem Flüssigkeitsverteiler (4), mit einem ersten Auslaß (7) für das Wasserstoffgas und mindestens einem zweiten Auslaß (8) für das hydrierte Produkt und mindestens einem Gas-Flüssigkeits-Abscheider (9) vorgesehen ist.

Fig. 1

## Verfahren zum Hydrieren von Fettsäureestern, Fetten, Fettsäuren und Vorrichtung zum Durchführen des Verfahrens

Die Erfindung betrifft ein Verfahren zum Hydrieren von Fettsäureestern, Fetten, Fettsäuren mit Wasserstoff in mindestens einem Festbrettreaktor.

In bekannten Verfahren zum Hydrieren von Fetten und Fettderivaten findet die Reaktion in der Sumpfphase mit suspendiertem Katalysator oder in Rieselfahrweise im Schüttreaktor über einen geformten Katalysator statt. Bei der Rieselfahrweise findet die eigentliche Hydrierreaktion in einem oder mehreren hintereinander geschalteten Festbettreaktoren bei Temperaturen von 200° bis 250° C und einem Wasserstoffdruck von 200 bis 300 bar statt. Dazu wird das Fett oder Fettderivat mit Presspumpen in die Anlage gedrückt, wo der zu hydrierende Stoff mit komprimiertem Wasserstoff vermischt, gemeinsam mit diesem auf Reaktionstemperatur erhitzt und von oben in den Reaktor geleitet wird. Dabei wird mit der Gleichstromfahrweise gearbeitet.

Da es sich bei dem Hydrieren um eine exotherme Reaktion handelt, wird, um die Wärme abzuführen und eine hohe Raumzeitausbeute zu erzielen, mit einem 50 bis 100fachen molaren Überschuß an Wasserstoff gearbeitet. Die Katalysatorbelastung beträgt 0,4 bis 0,6 Liter organischer Einsatz pro Stunde bezogen auf 1 Liter Katalysator (1/hl). Nach dem Durchlaufen durch den oder die Reaktoren wird das Reaktionsgemisch gekühlt und in einem Abscheider in die Flüssigphase und die Gasphase aufgetrennt.

Das bekannte Verfahren weist eine Reihe von reaktionstechnischen Nachteilen auf.

Da im Gleichstromverfahren gearbeitet wird, werden gasförmige und niedrig siedende Reaktionsprodukte wie Methanol bzw. Reaktionswasser, leichtsiedende Ester und Fettalkohole sowie Katalysatorgifte, z. B. flüchtige Halogen- und Schwefelverbindungen im Überschußwasserstoffstrom aufgenommen und über die gesamte Katalysatorstrecke geleitet. Durch die Katalysatorgifte wird der Katalysator relativ schnell desaktiviert.

Durch die verdampften Reaktionsprodukte wird das Verhältnis des Wasserstoffpartialdrucks zu den Partialdrücken der Endprodukte über den Reaktionsweg vom Kopf des Reaktors zum Produktaustritt hin verringert. Um eine hohe Raumzeitausbeute zu erhalten, muß mit hohem Wasserstoffüberschuß gefahren werden.

Zum Abtrennen der hydrierten Fettderivate, wie z. B. Fettalkoholen und Propandiol von den Nebenprodukten sind zusätzliche, aufwendige Verfahrensschritte notwendig. Dazu zählen der Methanol-, Propandiol- und Reaktionswasserabtrieb sowie die Alkoholabtrennung und Alkoholfraktionierung.

Diese Nachteile werden durch ein Gegenstromverfahren eliminiert. Der Anwendung eines solchen Verfahrens standen bisher jedoch folgende Bedenken entgegen: Da es sich um eine exotherme Reaktion handelt, könnten sich thermische Probleme ergeben. Bisher ging man nämlich davon aus, daß die Reaktionswärme durch einen hohen Überschuß an Wasserstoffgas aus den Reaktionszonen abgeführt werden muß. Im Gegenstromverfahren wäre unter dieser Voraussetzung ein solch hoher Überschuß an Wasserstoffgas notwendig, daß der Reaktor geflutet würde und eine sichere Betriebsweise des Reaktors nicht gewährleistet wäre. Durch die intensive Wechselwirkung zwischen Flüssigkeit, Gas und Katalysator ist im Gegenstromverfahren ein Auflockern der Schüttung und durch bewegte Katalysatortabletten deren Abrieb möglich. Der Katalysatorabrieb würde in diesem Fall die restliche Schüttung verstopfen und den Druckverlust erhöhen. Die Folge wäre das Fluten des Festbettes und eine unsichere Betriebsweise.

Aus diesen Gründen wurde bisher von einem Hydrieren von Fetten und Fettderivaten im Gegenstromverfahren abgesehen. Dadurch mußten aber die Nachteile des Gleichstromverfahrens in Kauf genommen werden.

Die Aufgabe der Erfindung liegt darin, ein Verfahren der eingangs genannten Art zu schaffen, das gegenüber dem Gleichstromhydrierverfahren eine größere Raumzeitausbeute, eine erhöhte Katalysatorstandzeit und eine Verringerung der Aufarbeitungsstufen aufweist.

Diese Aufgabe wird erfindungsgemäß dadurch gelöst, daß bei einem Verfahren der eingangs genannten Art im Gegenstrom hydriert wird, und daß die Stoffdurchflußraten und die Reaktorgeometrie so gewählt werden, daß ein Fluten des Reaktors nicht eintritt.

Im Gegensatz zu den bisher bekannten Bedenken gegen ein Gegenstromführen des Verfahrens treten nämlich überraschenderweise die erwarteten Probleme nicht auf. Es gibt keine thermischen Probleme, da durch die Verdampfung von Methanol bzw. Reaktionswasser genügend Wärme abgeführt wird, so daß die erwartete Notwendigkeit eines großen Wasserstoffgasüberschußes entfällt. Die Kühlung des exothermen Verfahrens wird also durch die Verdampfungswärme der verdampfbaren Reaktionsprodukte und eine bestimmte Formgebung des Reaktors oder durch eine externe Kühlung mit einem Kühlmedium erreicht.

Die erwartete hohe Katalysatordesaktivierung tritt, wie die Versuche zeigten, nicht auf, da die Stoff-

EP 0 350 763 A1

durchflußraten so gewählt werden können, daß ein Verwirbeln der Katalysatorpartikel nicht eintritt.

Im erfindungsgemäßen Verfahren führt die Gegenstromfahrweise zu einer Verbesserung des Stoffaustausches zwischen dem Gas, der Flüssigkeit und dem porösen Katalysator und damit zu einer höheren Raumzeitausbeute. Durch Versuche konnte gezeigt werden, daß dabei die optimalen Gasgeschwindigkeiten mit üblichen Katalysatorformlingen noch deutlich unter dem Flutpunkt liegen. Ein erhöhter Katalysatorabrieb wurde nicht beobachtet.

Im Gegensatz zum Gleichstromverfahren kommt beim Gegenstromverfahren am Ende des Hydrierreaktors nicht ein Wasserstoff-Nebenprodukt-Gemisch mit den fettchemischen Komponenten in Kontakt, sondern reiner Frischwasserstoff. Das Reaktionsgleichgewicht wird beim Gegenstromverfahren trotz geringerer Wasserstoffmenge in Richtung auf das Reaktionsprodukt Fettalkohol verschoben.

Mit dem erfindungsgemäßen Verfahren ist eine besonders vorteilhafte Verfahrensführung möglich. Dabei werden die Stoffdurchsätze, Temperaturen und Drücke so gewählt, daß die gasförmigen und leicht siedenden Reaktionsprodukte mit dem Wasserstoffstrom aus dem Reaktor geführt werden. Bei diesen Reaktionsprodukten handelt es sich insbesondere um Methanol bzw. Reaktionswasser sowie Vorlaufalkohol. Unter Vorlaufalkohol sind hier $C_6$- bis $C_{10}$-Fettalkohole zu verstehen. Dadurch ist es möglich, daß die mit dem Wasserstoffstrom aus dem Reaktor geführten Produkte zu einem separaten Hochdruckabscheider geführt werden. Im Gegensatz zum Gleichstromverfahren, in dem ein Trennverfahren für die leicht siedenden Stoffe nachgeschaltet werden muß, erfolgt bei dieser Betriebsweise der Leichtsiederabtrieb im Hydrierreaktor selbst. Vorteilhaft ist außerdem, daß der Trennschnitt leicht über die Wasserstoffgasmenge beeinflußt werden kann.

Ein weiterer Vorteil des erfindungsgemäßen Verfahrens besteht darin, daß flüchtige Reaktionsnebenprodukte, wie z. B. Methan, die das Reaktionsgleichgewicht zuungunsten verschieben, frühzeitig in die Gasphase übergehen und nur den Bereich zwischen der Reaktionszone und dem Wasserstoffaustritt, nicht aber den übrigen Bereich des Katalysators beeinträchtigen.

Durch das erfindungsgemäße Verfahren kann damit die Raumzeitausbeute und die Katalysatorstandzeit erhöht werden sowie die erste Aufarbeitungsstufe eingespart werden.

Ein zusätzlicher Vorteil des erfindungsgemäßen Gegenstromverfahrens besteht darin, daß durch die Wahl unterschiedlicher Temperaturen des Wasserstoffgases und des zu hydrierenden Einsatzes ein gewünschtes Temperaturprofil im Reaktor einstellbar ist. So kann vorgesehen werden, daß kurz nach der Haupt-Reaktionszone die Temperatur im Reaktor absinkt, so daß keine unerwünschten Folgeprodukte wie Kohlenwasserstoffe (KW) entstehen.

Besonders vorteilhaft ist es, wenn das Verfahren in einem im wesentlichen senkrechten Reaktor durchgeführt wird, wobei das Wasserstoffgas in der Nähe des unteren Endes des Reaktors in diese einströmt. Vorzugsweise tritt dabei der zu hydrierende Einsatz im oberen Drittel der Katalysatorzone in den Reaktor ein oder die Gasphase wird durch einen nachgeschalteten Festbettreaktor geführt. In dieser Ausgestaltung strömen die leichter flüchtigen Einsatzstoffe durch einen Teil der Katalysatorzone bzw. durch den nachgeschalteten Festbettreaktor und werden auf diese Weise vollständig hydriert.

Vorteilhaft ist ferner, wenn als Reaktor ein Festbettreaktor mit hohem Schlankheitsgrad eingesetzt wird. In ihm kann eine hohe Raumzeitausbeute erzielt und über eine Außenkühlung die Reaktionswärme gut abgeführt werden. Stattdessen oder zusätzlich kann die Reaktionswärme auch dadurch abgeführt werden, daß ein Reaktor mit einem indirekten Kühler verwendet wird.

Die Erfindung betrifft auch eine Vorrichtung zum Durchführen des eingangs genannten Verfahrens. Diese Vorrichtung weist mindestens einen Hydrierreaktor mit mindestens einem ersten Einlaß für Wasserstoff und einem Gasverteiler, mindestens einem zweiten Einlaß für den zu hydrierenden Einsatz mit einem Flüssigkeitsverteiler, mindestens einem ersten Auslaß für das Wasserstoffgas und mindestens einem zweiten Auslaß für das hydrierte Produkt sowie mindestens einen Gas-Flüssigkeits-Abscheider auf. Charakteristisch für die Vorrichtung ist, daß mindestens zwei Einlässe und mindestens zwei Auslässe erforderlich sind.

Die Erfindung wird nachstehend an Hand von Ausführungsbeispielen unter Bezugnahme auf die Zeichnung näher erläutert.

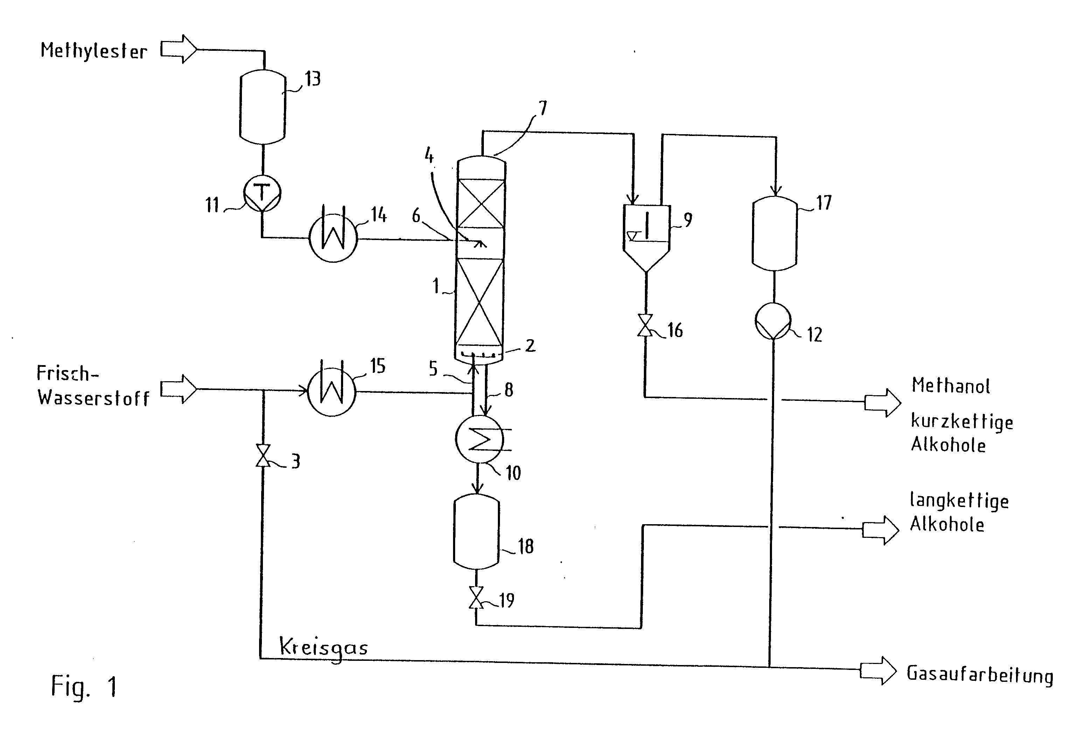

Die Figur zeigt schematisch eine besonders bevorzugte Ausführungsform der erfindungsgemäßen Vorrichtung. Es wird am Beispiel der Hydrierung von Fettsäuremethylester näher erläutert. Figur 1 zeigt einen Festbettreaktor 1 mit einer Katalysatorfüllung, der mit einem Gas-Flüssigkeits-Abscheider 9 und einem Produktkühler 10 verbunden ist.

Erwärmter Fettsäuremethylester wird aus der Vorlage 13 von einer Dosierpumpe 11 durch einen Erhitzer 14 zum zweiten Einlaß 6 am Kopf des senkrecht stehenden Reaktors 1 gedrückt und dort durch den Flüssigkeitsverteiler 4. Wasserstoffgas wird von einer Gaspumpe 12 über ein Kreisgasregelventil 3 durch einen Wasserstoff-Erhitzer 15 zum Fuß des Reaktors 1, zu einem ersten Einlaß 5 am unteren Ende

3

des Reaktors 1 und dort durch den Gasverteiler 2 gepreßt. Das am ersten Auslaß 7, welcher sich am Kopf des Reaktors 1 befindet, herausströmende Wasserstoffgas, das verdampftes Methanol und leicht siedende Fettalkohole mit sich führt, wird in einen Gas-Flüssigkeits-Abscheider 9 geleitet. Dort findet eine Trennung in Flüssig- und Gasphase statt. Die Gasphase wird über eine Vorlage 17 zurück zur Gaspumpe 12 geführt. Die Flüssigphase aus Abscheider 9 wird über ein Ablaßventil 16 zur Methanolabtrennung geleitet. An einem zweiten Auslaß 8 des Reaktors 1 werden die höher siedenden Fettalkohole über einen Produktkühler 10 in eine Produktvorlage 18 für langkettige Alkohole mit einem Ablaßventil 19 geführt.

Im folgenden sind die Ergebnisse von Hydrierversuchen in einer 4-Liter-Gegenstromhydrieranlage mit den zu hydrierenden Produkten $C_{12/18}$-Methylester, $C_{12/18}$-Fettsäure und raffiniertem Kokosöl tabellarisch zusammengefaßt.

Beispiele

1. Beispiel

| Hydrierung von Fettsäuremethylester $C_{12/18}$ (ME) | | |
|---|---|---|
| | Katalysator: | |
| | handelsüblicher Hydrierkatalysator | |
| | 3 mm Tablette | |
| 1) | Reaktionsbedingungen: | |
| | Druck:<br>Temperatur Reaktor oben:<br>Temperatur Reaktor unten:<br>$H_2$-Durchsatz:<br>Einsatz $C_{12/18}$-ME | 250 bar<br>230 °C<br>202 °C<br>1,5 Nm³/l kat. h<br>1 l/l kat. h |
| 2) | Produkt: | |
| | Produktvorlage 18 | |
| | Fettalkohol:<br>OH-Zahl (OHZ):<br>% KW:<br>Verseifungszahl (VZ): | 3,31 l/h<br>270<br>kleiner 1,0<br>0,8 |
| | Abscheider 9 | |
| | Methanol:<br>Fettalkohol und nicht umgesetzter Ester: | 0,54 l/h<br>0,24 l/h |

4

## 2. Beispiel

| Änderung der Gasphasenbelastung | | | | |
|---|---|---|---|---|
| Reaktionsbedingung: | | | | |
| P = 250 bar, T = 230 °C, LHSV in l/l kat. h sonst wie beim 1. Beispiel | | | | |
| Gasdurchsatz (Nm³/h) | GHSV (Nl/l kat. h) | Umsatz (%) | Ausbeute (%) | Selektivität (%) |
| 2 | 500 | 99,0 | 90,9 | 91,8 |
| 4 | 1.000 | 99,5 | 98,6 | 99,1 |
| 6 | 1.500 | 99,7 | 99,3 | 99,6 |
| 8 | 2.000 | 99,7 | 98,9 | 99,2 |
| 10 | 2.500 | 99,6 | 98,6 | 99,0 |
| 15 | 3.750 | 99,1 | 98,6 | 99,5 |
| Erst bei einer GHSV kleiner 1.000 l/l h treten aufgrund von lokaler Exothermie erhörte Werte der KW-Bildung (etwa 8 %) auf. | | | | |

## 3. Beispiel

| Einfluß der Hydrierparameter Druck, Gas- und Flüssigphasenbelastung auf die Ausbeute und Selektivität bei der Hydrierung von C$^{12/18}$-Methylester (Katalysator wie in Beispiel 1) | | | |
|---|---|---|---|
| Ergebnisse aus Vorlage 18 | | | |
| 1) Druckeinfluß | | | |
| (LHSV in l/l kat. h, T = 230 °C, GHSV = 1.500 Nl/l kat. h) | | | |
| Druck (bar) | Umsatz (ME) (%) | Ausbeute (FA) (%) | Selektivität (FA) (%) |
| 250 | 99,6 | 99,4 | 99,8 |
| 200 | 99,3 | 97,6 | 98,3 |
| 150 | 98,8 | 96,8 | 98,0 |
| 100 | 97,2 | 95,0 | 97,7 |
| 50 | 84,6 | 74,9 | 88,5 |
| FA = Fettalkohol | | | |
| 2) Flüssigphasenbelastung | | | |
| (P = 250 bar, T = 230 °C, GHSV = 1.500 Nl/l kat. h) | | | |
| LHSV (l/l h) | Umsatz (ME) (%) | Ausbeute (FA) (%) | Selektivität (FA) (%) |
| 0,75 | 99,7 | 99,8 | 100 |
| 1,0 | 99,6 | 99,4 | 99,8 |
| 1,25 | 99,5 | 97,9 | 98,4 |
| 1,5 | 99,4 | 94,3 | 94,9 |

5

## 4. Beispiel

| Hydrierung von $C_{12/18}$-Fettsäure | |
|---|---|
| Katalysator: | |
| Cu-Chromit, saürestabil<br>3 mm Extrudat | |
| Reaktionsbedingungen: | |
| $H_2$-Druck:<br>Temperatur Reaktor oben:<br>Temperatur Reaktor unten:<br>$H_2$-Durchsatz:<br>Einsatz $C_{12/18}$-Fettsäure: | 250 bar<br>250 bis 300 °C<br>230 bis 270 °C<br>1,75 Nm³/l h<br>0,5 l/l h |
| Produkt: | |
| Vorlage 18:<br><br><br><br><br>Abscheider 9: | $C_{12/18}$-Hauptlaufalkohol<br>OHZ größer 255<br>SZ kleiner 0,5<br>VZ kleiner 2<br>KW kleiner 2 %<br>($H_2$-Überschußgas)<br>0,23 l/h Reaktionswasser<br>0,17 l/h Vorlauffettalkohol |

## 5. Beispiel

| Hydrierung von raffiniertem Kokosöl | |
|---|---|
| Katalysator: | |
| handelsüblicher Hydrierkatalysator<br>3 mm Tablette | |
| Reaktionsbedingungen: | |
| Druck:<br>Temperatur Reaktor oben:<br>Temperatur Reaktor unten:<br>$H_2$-Durchsatz:<br>Kokosöl, raff. Einsatz: | 250 bar<br>240 °C<br>195 °C<br>1,5 Nm³/l h<br>1,0 l/l h |
| Produkt: | |
| Vorlage 18:<br><br><br>Abscheider 9: | Hauptlaufalkohol, $C_3$-diolfrei<br>VZ = 2,5<br>OHZ = 195<br>($H_2$-Überschußgas)<br>220 ml/h $H_2O$ mit 14 g/l $C_3$-Diol<br>460 ml/h $C_6$-$C_{10}$-Fettalkohol |

## Bezugszeichenliste

1 = Reaktor, Festbettreaktor

2 = Gasverteiler

3 = Kreisgasregelventil

4 = Flüssigkeitsverteiler

5 = erster Einlaß

6 = zweiter Einlaß

7 = erster Auslaß

8 = zweiter Auslaß

9 = Gas-Flüssigkeits-Abscheider

10 = Produktkühler

11 = Dosierpumpe (für Fettrohstoff)

12 = Gaspumpe

13 = Vorlage (für Fettrohstoff)

14 = Erhitzer (für Fettrohstoff)

15 = Wasserstoff-Erhitzer

16 = Ablaßventil

17 = Vorlage

18 = Produktvorlage

19 = Ablaßventil

**Ansprüche**

1. Verfahren zum Hydrieren von Fettsäureestern, Fetten, Fettsäuren mit Wasserstoff in mindestens einem Festbettreaktor,
dadurch gekennzeichnet,
daß im Gegenstrom hydriert wird und daß die Stoffdurchflußraten und die Reaktorgeometrie so gewählt werden, daß ein Fluten des Reaktors (1) nicht eintritt.

2. Verfahren nach Anspruch 1, dadurch gekennzeichnet,
daß die Stoffdurchflußraten so gewählt werden, daß ein Verwirbeln der Katalysatorpartikel nicht eintritt.

3. Verfahren nach Anspruch 1 oder 2, dadurch gekennzeichnet,
daß die Stoffdurchsätze, Temperaturen und Drücke so gewählt werden, daß die gasförmigen und leichtsiedenden Reaktionsprodukte mit dem Wasserstoffstrom aus dem Reaktor (1) geführt werden.

4. Verfahren nach einem der Ansprüche 1 bis 3, dadurch gekennzeichnet,
daß die Temperaturen des Wasserstoffgases und des zu hydrierenden Einsatzes entsprechend einem gewünschten Temperaturprofil im Reaktor eingestellt werden.

5. Verfahren nach einem der Ansprüche 1 bis 4, dadurch gekennzeichnet,
daß es in einem im wesentlichen senkrechten Reaktor (1) durchgeführt wird, wobei das Wasserstoffgas in der Nähe des unteren Endes (2) des Reaktors (1) in diesen einströmt.

6. Verfahren nach einem der Ansprüche 1 bis 5, dadurch gekennzeichnet,
daß der zu hydrierende Einsatz im oberen Drittel der Katalysatorzone in den Reaktor (1) eintritt oder die Gasphase durch einen nachgeschalteten Festbettreaktor geführt wird.

7. Verfahren nach einem der Ansprüche 1 bis 6, dadurch gekennzeichnet,
daß als Reaktor (1) ein Festbettreaktor mit hohem Schlankheitsgrad eingesetzt wird.

8. Verfahren nach einem der Ansprüche 1 bis 7, dadurch gekennzeichnet,
daß ein Reaktor (1) mit einem indirekten Kühler verwendet wird.

9. Vorrichtung zum Durchführen des Verfahrens nach einem der Ansprüche 1 bis 6, gekennzeichnet durch
mindestens einen Hydrierreaktor (1) mit mindestens einem ersten Einlaß (5) für Wasserstoff und einem Gasverteiler (2), mit mindestens einem zweiten Einlaß (6) für den zu hydrierenden Einsatz mit einem Flüssigkeitsverteiler (4), mit mindestens einem ersten Auslaß (7) für das Wasserstoffgas und mindestens einem zweiten Auslaß (8) für das hydrierte Produkt und mindestens einem Gas-Flüssigkeits-Abscheider (9).

Fig. 1

Methylester

Frisch-
Wasserstoff

Kreisgas

Methanol

kurzkettige
Alkohole

langkettige
Alkohole

Gasaufarbeitung

EP 0 350 763 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-1 343 255 (CARLETON ELLIS) * Seite 2, Zeile 114 - Seite 3, Zeile 9; Seite 3, Zeilen 80-120; Ansprüche; Figur 1 * | 1,2,4-6 ,8 | C 11 C 3/12 |
| Y | --- | 3,7,9 | |
| Y | LU-A- 70 819 (STUDIENGESELLSCHAFT KOHLE mbH) * Beispiele 1-3; Seiten 3,8,9; Figuren * | 3,7,9 | |
| X | --- CHEMICAL ABSTRACTS, Band 73, Nr. 20, 16. November 1970, Seite 84, Zusammenfassung Nr. 100279y, Columbus, Ohio, US; V.I. KOMAROV: "Fat hydrogenation on fixed nickel-chromium catalysts by various methods", & IZV. UCHEB. ZAVED., PISHCH. TEKHNOL. 1970, (3), 83-7 * Zusammenfassung * | 1,2,5 | |
| A | --- GB-A-1 016 583 (UNILEVER LTD) * Seite 2, Zeilen 97-104; Beispiel 1 * --- | 1,2 | RECHERCHIERTE SACHGEBIETE (Int. Cl.5) C 11 C |
| A | US-A-3 160 579 (J. VAN DYCK) --- | | |
| A | EP-A-0 230 971 (HENKEL KG) ----- | | |

Der vorliegende Recherchenbericht wurde für alle Patentansprüche erstellt

| Recherchenort | Abschlußdatum der Recherche | Prüfer |
|---|---|---|
| DEN HAAG | 22-09-1989 | LEPRETRE F.G.M.J. |